(19) Europäisches Patentamt
European Patent Office
Office européen des brevets

(11) Veröffentlichungsnummer: **0 315 861 B1**

# (12) EUROPÄISCHE PATENTSCHRIFT

(45) Veröffentlichungstag der Patentschrift: **29.01.92**

(51) Int. Cl.5: **A61K 31/71**, //(A61K31/71, 31:41),(A61K31/71,31:495)

(21) Anmeldenummer: **88118101.0**

(22) Anmeldetag: **31.10.88**

## (54) Antimycotische Zusammensetzungen aus Nikkomycin-Verbindungen und antimycotischem Azol.

(30) Priorität: **09.11.87 US 118078**

(43) Veröffentlichungstag der Anmeldung:
**17.05.89 Patentblatt 89/20**

(45) Bekanntmachung des Hinweises auf die Patenterteilung:
**29.01.92 Patentblatt 92/05**

(84) Benannte Vertragsstaaten:
**AT BE CH DE ES FR GB GR IT LI NL SE**

(56) Entgegenhaltungen:
**US-A- 4 158 608**
**US-A- 4 315 922**
**US-A- 4 381 306**

(73) Patentinhaber: **BAYER AG**

**W-5090 Leverkusen 1 Bayerwerk(DE)**

(72) Erfinder: **Schaller, Klaus, Dr.**
**Am Sonnenschein 38**
**W-5600 Wuppertal 1(DE)**
Erfinder: **Moeschler, Heinrich Ferdinand, Dr.**
**Woodwind Condominiums 613 Clarinet Blvd. East**
**Elkhart, IN 56516(US)**
Erfinder: **Plempel, Manfred, Dr.**
**Zwengenberger Strasse 3c**
**W-5657 Haan(DE)**
Erfinder: **Hector, Richard F., Dr.**
**7891 Jade Circle**
**Dublin California 94568(US)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft antimykotische Zusammensetzungen, die fungizid wirksame Mengen eines Nikkomycins und ein antimykotisches Azol umfassen, die Herstellung solcher Zusammensetzungen und Verfahren zur Herstellung von Mitteln zur Behandlung von Mykosen.

Über Verbindungen, die die Synthese des fungalen Zellwandmaterials inhibieren (Synthaseinhibitoren), wurde kürzlich berichtet, daß sie bemerkenswerte Wirkungen gegenüber Pilzen besitzen, die von landwirtschaftlicher Bedeutung sind (US-B- 4 315 922 und 4 158 608; US-B- 4 585 761 und 4 552 954 hinsichtlich der Beschreibung der Herstellung und Reinigung solcher Verbindungen). Von den in den aufgeführten Patentschriften genannten Verbindungen, den Nikkomycinen, ist bekannt, daß sie zusammen mit ähnlichen Verbindungen, bekannt als Polyoxine, durch Beeinträchtigung der Synthese von Chitin in den Zellwänden von Pilzen wirken. Da die Pilze, die von medizinischer Bedeutung für die Menschen sind, auch variierende Mengen von Chitin in ihren Zellwänden haben, wurden Experimente durchgeführt, um zu bestimmen, ob die Chitin-Synthaseinhibitoren auch in der Lage sind, das Wachstum solcher Pilze zu hemmen (Hector und Pappagianis, J. Bacteriol. 154:488-498, 1983, und Hector und Braun, Antimicrobial Agents Chemother., 29:389-394(1986). In früheren Arbeiten wurde über bestimmte Pilze, wie Candida albicans, berichtet, daß sie gegenüber Chitin-Synthaseinhibitoren unempfindlich seien (Naider et al; Anitimicrobial Agents Chemother. 24: 787-796, 1983). Später wurde gefunden, daß C. albicans gegenüber Nikkomycinen empfindlicher sind als gegenüber Polyoxinen (Yadan et al; J. Bacteriol. 160:884-888, 1984), aber die Konzentrationen, die zum Abtöten dieser Hefe notwendig sind, würden (aus Toxizitätsgründen) ihre Anwendung als chemotherapeutische Mittel für Hefeinfektionen ausschließen (Hector und Braun, Antimicrobial Agents Chemother., 29:389-394,1986).

Es wurde nun überraschend gefunden, daß bestimmte Nikkomycinverbindungen in Kombination mit bestimmten antimykotisch wirksamen Azolen bei der Behandlung fungaler Infektionen wirksam sind.

Wie in synergetischen Studien gezeigt wurde, ist die erreichte antimykotische Wirkung ein synergistischer Effekt, der auf der Kombination dieser Nikkomycine mit diesen Azolen beruht.

Dementsprechend betrifft die Erfindung eine antimykotische Zusammensetzung, die eine antimykotisch wirksame Menge dieser Nikkomycine und dieser Azole umfaßt.

Nikkomycine und antimykotisch wirksame Azole sind allgemein in GB-B- 1 351 542, CA-B- 925 504, CA-B- 946 391, AU-B- 542 110, AU-B- 551 411, US-B- 4 301 166, US-B- 4 381 306, US-B- 4 246 274, US-B-4 238 498, US-B- 4 207 328, US-B- 3 968 229 und DE-A 3 242 249 beschrieben. Bei den Nikkomycinen der Erfindung handelt es sich um Nikkomycin X der Formel I sowie Nikkomycin Z der Formel II

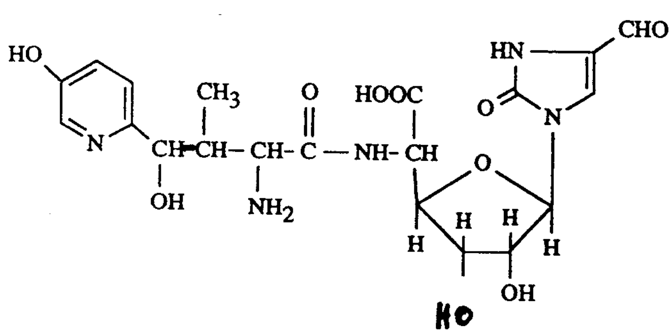

(I)

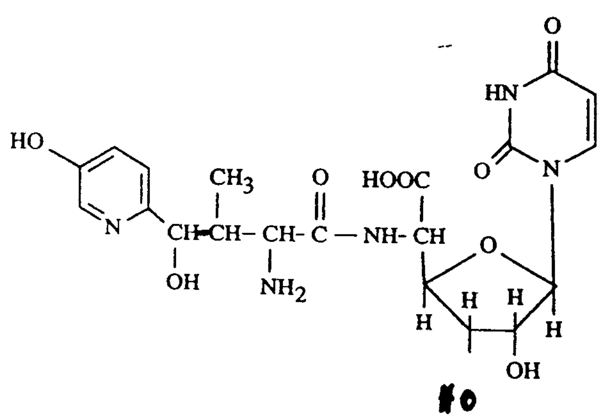

(II)

Bei den antimykotisch wirksamen Azolderivaten der Erfindung handelt es sich um 1-(4-Chlorphenyl)-2-methyl-2-methoximinomethyl-1-(1,2,4-triazol-1-yl-methyl)-1-propanol (R 3783) der Formel III

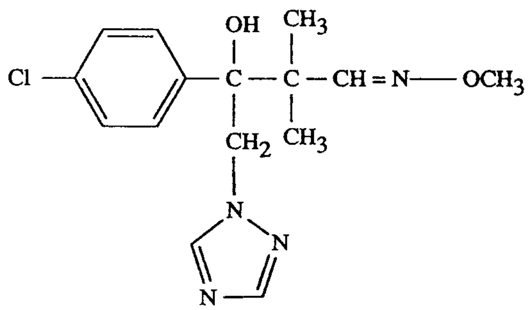
(III)

1-(4-Chlorphenoxy)-3,3-dimethyl-2-(1,2,4-triazol-1-yl-methyl)-butan-2-ol (VIBUNAZOL; N 7133) der Formel IV

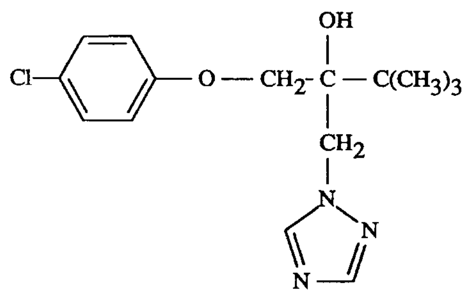
(IV)

1-(4-Fluorphenyl)-1-(1-methylsulfinyl-1-cyclopropyl)-(1,2,4-triazol-1-yl-methyl)-methanol (U 7476) der Formel V

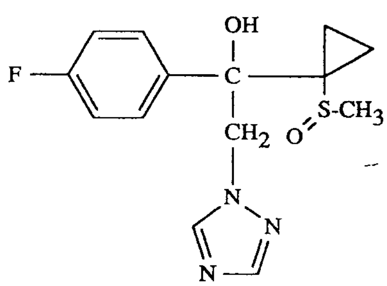
(V)

Miconazol der Formel VI

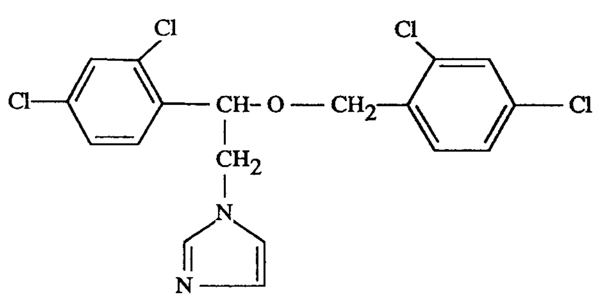
(VI)

Ketoconazol der Formel VII

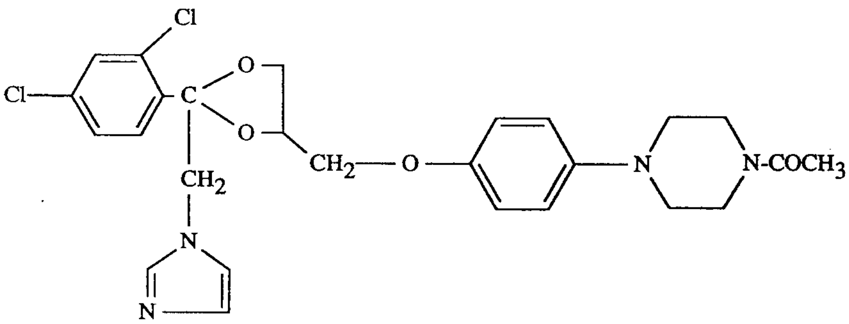

(VII)

Itraconazol der Formel VIII

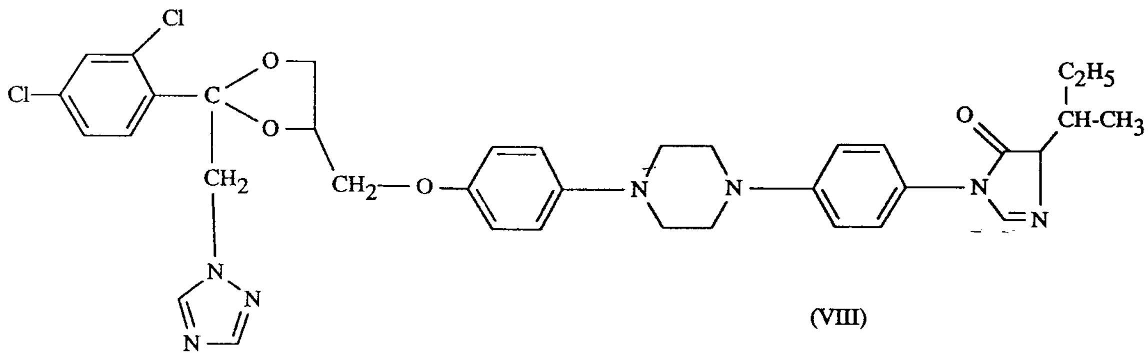

(VIII)

Fluconazol der Formel IX

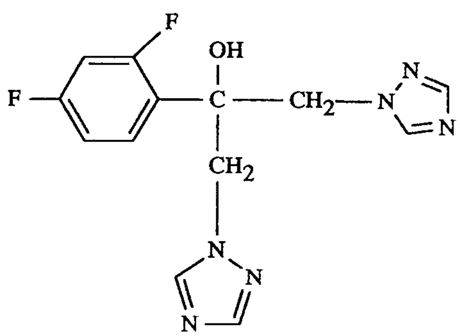

(IX)

Clotrimazol der Formel X

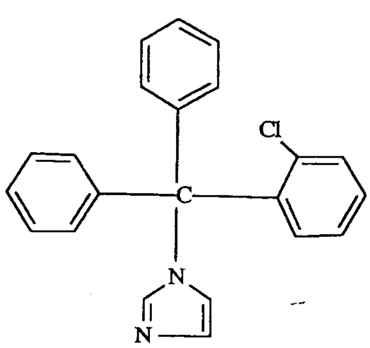

(X)

sowie Bifonazol der Formel XI

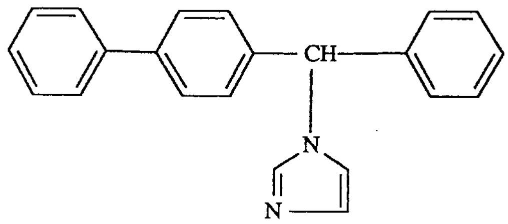

(XI)

sowie Additionsprodukte von Säuren mit diesen Azolderivaten.

Bevorzugt sind Zusammensetzungen, die als Nikkomycin-Komponente das Nikkomycin Z der Formel II enthalten.

Säuren, die zur Salzbildung genommen werden können, sind Halogenwasserstoffsäuren, wie z.B. Salzsäure und Bromwasserstoffsäure, insbesondere Salzsäure, Phosphorsäure, Salpetersäure, monofunktionale und bifunktionale Carbonsäuren und Hydroxycarbonsäuren, wie z. B. Essigsäure, Maleinsäure, Bernsteinsäure, Fumarsäure, Weinsäure, Citronensäure, Salicylsäure, Sorbinsäure und Milchsäure, und Sulfonsäuren, wie o-Toluolsulfonsäure und 1,5-Naphthalindisulfonsäure.

Die antimykotischen Zusammensetzungen gemäß der Erfindung enthalten eine fungizidal wirksame Menge des Nikkomycins und ein Azol in einem Verhältnis von 1:1 bis 30:1, vorzugsweise 3:1 bis 10:1, wobei die Verabreichung von 1 mg/kg bis 1000 mg/kg Körpergewicht pro Tag, vorzugsweise von 10 mg/kg bis 100 mg/kg, eines Nikkomycins und eines antimykotischen Azols der oben identifizierten Art vorgesehen ist.

Die Erfindung betrifft auch die Verwendung von Nikkomycinen in Kombination mit antimykotisch wirksamen und gegebenenfalls oral anwendbaren Azolen bei der Herstellung von antimikotisch wirksamen, pharmazeutischen Zusammensetzungen, die das Mischen einer Kombination einer antimykotisch wirksamen Menge eines Nikkomycins und eines Azols mit einem festen oder flüssigen Streckmittel und/oder Träger oder anderen, für die Herstellung von pharmazeutischen Zusammensetzungen geeigneten Hilfsmitteln, wobei das Nikkomycin und das Azol vorzugsweise in einem Verhältnis von 1:1 bis 30:1 vorliegen, umfaßt.

Vorzugweise werden die Zusammensetzungen der Erfindung oral verabreicht, aber sie können auch parenteral oder über Inhalation eines aerosilisierten Präparates verabreicht werden.

Die Zusammensetzungen gemäß der Erfindung besitzen ein sehr breites antimykotisches Wirkungsspektrum speziell gegen Dermatophyten und Hefen als auch biphasische Pilze, z. B. gegen eine Vielzahl von Candida, wie Candida albicans, eine Vielzahl von Epidermophyton Floccusum, eine Vielzahl von Aspergillus, wie Aspergillus niger und Aspergillus fumigatus, eine Vielzahl von Trichophyton, wie Trichophyton mentagrophytes, eine Vielzahl von Microsporon, wie Microsporon felineum, und eine Vielzahl von Torulopsis, wie Torulopsis glabrata, und Coccidioides immitis, Histoplasma capsulatum und Blastomyces dermatitidis. Die Aufführung dieser Mikroorganismen beinhaltet in keiner Weise eine Beschränkung der Keime, die bekämpft werden können, sondern dient allein Illustrationszwecken.

Beispiele für die Indikationsgebiete in der Humanmedizin sind: Dermatomycosen und systemische Mycosen, verursacht durch Trichophyton mentagrophytes und andere Variationen von Trichophyton , Variationen von Microsporon, Epidermophyton floccusum, Hefen und biphasischen Pilzen als auch sonstigen Pilzen.

Beispiele für Indikationsgebiete in der Tiermedizin sind alle Dermatomycosen und systemischen Mycosen, insbesondere solche, die durch die oben genannten Keime verursacht sind.

Die Erfindung beinhaltet pharmazeutische Formulierungen, die zusätzlich zu nicht-toxischen, inerten, pharmazeutisch geeigneten Arzneimittelträgern eine oder mehrere wirksame Verbindungen gemäß der Erfindung enthalten oder die aus einer oder mehreren aktiven Verbindungen gemäß der Erfindung bestehen, als auch Verfahren zur Herstellung dieser Formulierungen.

Die Erfindung beinhaltet auch pharmazeutische Formulierungen und Dosierungseinheiten. Dies bedeutet, daß die Formulierungen in Form von einzelnen Teilen, z.B. Tabletten, Dragees, Kapseln, Pillen, Zäpfchen und Ampullen vorliegen, deren Gehalt an Wirkkomponente einem Teil oder einem Vielfachen einer einzelnen Dosis entspricht. Diese Dosierungseinheiten können z.B. ein, zwei, drei oder vier Einzeldosierungen oder 1/2, 1/3 oder 1/4 einer einzelnen Dosierung enthalten. Eine einzelne Dosierung enthält vorzugsweise die Menge an Wirksubstanz, die in einer Verabreichung gegeben ist und die gewöhnlich einem ganzen Teil, einer Hälfte, einem Drittel oder einem Viertel einer täglichen Dosierung entspricht.

Unter nicht-toxischen, inerten, pharmazeutisch geeigneten Arzneimittelträgern sind feste, halbfeste oder flüssige Streckmittel, Füller und Hilfsformulierungen jeglicher Art zu verstehen.

Tabletten, Dragees, Kapseln, Pillen, Granulate, Zäpfchen, Lösungen, Suspensionen und Emulsionen, Pasten, Salben, Gele, Cremelotions, Pulver und Sprays können als bevorzugte pharmazeutische Formulierungen genannt werden.

Tabletten, Dragees, Kapseln, Pillen und Granulate können die Wirkkomponente oder Wirkkomponenten neben den üblichen Arzneimittelträgern, wie (a) Füller und Streckmittel, z.B. Stärke, Lactose, Succrose, Glucose, Mannitol und Silica; (b) Binder, z.B. Carboxymethycellulose, Alginate, Gelatine und Polyvinylpyrrolidon; (c) Anfeuchter, z.B. Glycerol; (d) Trennmittel, z.B. Agar-Agar, Calciumcarbonat und Natriumcarbonat; (e) Verzögererlösungen, z. B. Paraffin; und (f) Resorptionsbeschleuniger, z.B. Cetylalkohol und Glycerolmonostearat; (g) Adsorbentien, z.B. Kaolin und Bentonit; und (h) Schmiermittel, z.B. Talk, Calciumstearat und Magnesiumstearat und feste Polyethylenglykole; oder Mischungen der unter (a) bis (h) aufgeführten Substanzen enthalten.

Die Tabletten, Dragees, Kapseln, Pillen und Granulate können mit üblichen Überzügen und Umhüllungen versehen werden, die gegebenenfalls Opakierungsmittel enthalten und auch so zusammengesetzt sein können, daß sie die Wirkkomponente oder -komponenten alleine oder bevorzugt in einem gewissen Teil des Verdauungstraktes, gegebenenfalls in verzögerter Weise, freisetzen, wobei Beispiele für Einbettungszusammensetzungen, die verwendet werden können, Polymersubstanzen und Wachse sind.

Die Wirkkomponente oder -komponenten können auch, gegebenenfalls zusammen mit einem oder mehreren der oben genannten Arzneimittelträger, in einer mikroverkapselten Form vorliegen.

Die Zäpfchen können zusätzlich zu der Wirkkomponente oder -komponenten die üblichen wasserlöslichen oder wasserunlöslichen Arzneimittelträger enthalten, z.B. Polyethylenglykol, Fette, z.B. Kakaofett, höhere Ester (z.B. $C_{14}$-Alkohol mit $C_{16}$-Fettsäure), oder Mischungen dieser Substanzen.

Salben, Pasten, Cremes und Gele können zusätzlich zu der Wirkkomponente oder -komponenten, die üblichen Arzneimittelträger, z.B. tierische und pflanzliche Fette, Wachse, Paraffine, Stärke, Tragacanth, Cellulosederivate, Polyethylenglykole, Silicone, Bentonite, Silica, Talk und Zinkoxid, oder Mischungen dieser Substanzen, enthalten.

Pulver und Sprays können zusätzlich zu der Wirkkomponente oder -komponenten die üblichen Arzneimittelträger, z.B. Lactose, Talk, Silica, Aluminiumhydroxid, Calciumsilicat und Polyamidpulver oder Mischungen dieser Substanzen enthalten. Sprays können zusätzlich die üblichen Treibmittel, z.B. Chlorfluorkohlenwasserstoffe, enthalten.

Lösungen und Emulsionen können zusätzlich zu der Wirkkomponente oder -komponenten die üblichen Arzneimittelträger, wie Lösungsmittel, Lösungshilfsmittel und Emulgiermittel, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Weizenkeimöl, Erdnußöl, Maiskeimöl, Olivenöl, Castoröl und Sesamöl, Glycerol-Formal, Tetrahydrofurfurylalkohol, Polyethylenglykole und Fettsäureester von Sorbitan, oder Mischungen dieser Substanzen enthalten.

Zur parenteralen Verabreichung können die Lösungen und Emulsionen auch in einer sterilen Form, die isotonisch mit Blut ist, vorliegen.

Die Suspensionen können zusätzlich zu der Wirkkomponente oder -komponenten die üblichen Arzneimittelträger, wie flüssige Streckmittel, z.B. Wasser, Ethylalkohol oder Propylenglykol, Suspendiermittel, z.B. ethoxylierte Isostearylalkohole, Polyoxyethylensorbitolester und Sorbitanester, mikrokristalline Cellulose, Aluminiummetahydroxid, Bentonite, Agar-Agar und Tragacanth, oder Mischungen dieser Substanzen enthalten.

Die erwähnten Formulierungsformen können auch Farbmittel, Konservierungsmittel und Additive enthalten, die den Geruch und Geschmack verbessern, z.B. Pfefferminzöl und Eukalyptusöl, und Süssungsmittel, z.B. Sacharin.

Die therapeutisch wirksamen Verbindungen sollten vorzugsweise in den oben genannten pharmazeutischen Formulierungen in einer Konzentration von ungefähr 0,1 bis 99,5, vorzugsweise von ungefähr 0,5 bis 95 Gew.%, in bezug auf die Gesamtmischung, vorliegen.

Die oben genannten pharmazeutischen Formulierungen können auch andere pharmazeutische Wirkstoffe zusätzlich zu den Wirkstoffen gemäß der Erfindung enthalten.

Die oben genannten pharmazeutischen Formulierungen werden in üblicher Weise hergestellt, z.B. durch Mischen der Wirkkomponente oder -komponenten mit dem Arzneimittelträger oder Arzneimittelträgern.

Die Wirkstoffe oder die pharmazeutischen Formulierungen können lokal, oral in aerosillsierter Form, parenteral und insbesondere intravenös verabreicht werden.

Im allgemeinen hat es sich als günstig sowohl in der Humanmedizin als auch in der Tiermedizin erwiesen, die Wirkkomponente oder -komponenten gemäß der Erfindung in Gesamtmengen von ungefähr 10 bis 300, vorzugsweise 50 bis 200 mg/kg Körpergewicht alle 24 Stunden, gegebenenfalls in Form von mehreren, einzelnen Verabreichungen, zu verabreichen, um die gewünschten Ergebnisse zu erzielen.

Jedoch kann es notwendig sein, von den genannten Dosierungen abzuweichen, und dies wird insbesondere in Abhängigkeit von der Spezies und dem Körpergewicht des zu behandelnden Subjektes, der Art und Schwere der Krankheit, der Art der Formulierung und der Verabreichung des Arzneimittels und der Zeit oder des Intervalls, über die die Verabreichung vorgesehen ist, erfolgen. Somit kann es in manchen Fällen ausreichen, weniger als die oben genannten Mengen an Wirkstoff anzuwenden, während in anderen Fällen die oben genannte Menge des Wirkstoffs überschritten werden muß. Das Optimum und die Art der Verabreichung der Wirkstoffe können leicht durch jeden Fachmann bestimmt werden.

Die Fig. 1 bis 9 zeigen synergetische Studien für erfindungsgemäße, antifungal wirkende Nikkomycin-Azol-Zusammensetzungen.

Um die Existenz einer synergistischen Wirkung von Nikkomycinen und Azolen zu demonstrieren, wurden randomisierte in vitro-Versuche mit der Hefe Candida albicans durchgeführt.

Beispiele

Candida albicans B311 wurde über Nacht in einer Glucose-Hefeextrakt-Kulturgezüchtet, gewaschen und auf die gewünschte Dichte gebracht.

Die Tests wurden in 96-Loch-Mikrotiterplatten durchgeführt, wobei man eine Hefestickstoffkultur mit Dextrose und Asparagin als Wachstumsmedium verwendete. Die Testmaterialien wurden gelöst, serienmäßig verdünnt und dann in die geeigneten Löcher der Mikrotiterplatten überführt, so daß die Endkonzentrationen von 0,02 bis 1 mMol für die Nikkomycine, und 0,06 bis 64 µg für die Azole betrugen. Alle Löcher wurden mit 10.000 Organismen/ml (Endkonzentration) geimpft und die Platten bei Raumtemperatur 48 Stunden lang inkubiert. Die Platten wurden dann ausgewertet und die Wachstumsrate für jedes Loch festgestellt. Die Endpunkte wurden für jede Reihe als niedrigste Konzentration, die eine vollständige Inhibierung des Wachstums zeigte, bestimmt. Die Daten wurden als Isobologramme geplottet, die die Endpunkte für die einzelnen Verbindungen als auch die Endpunkte für die verschiedenen Kombinationen zeigten.

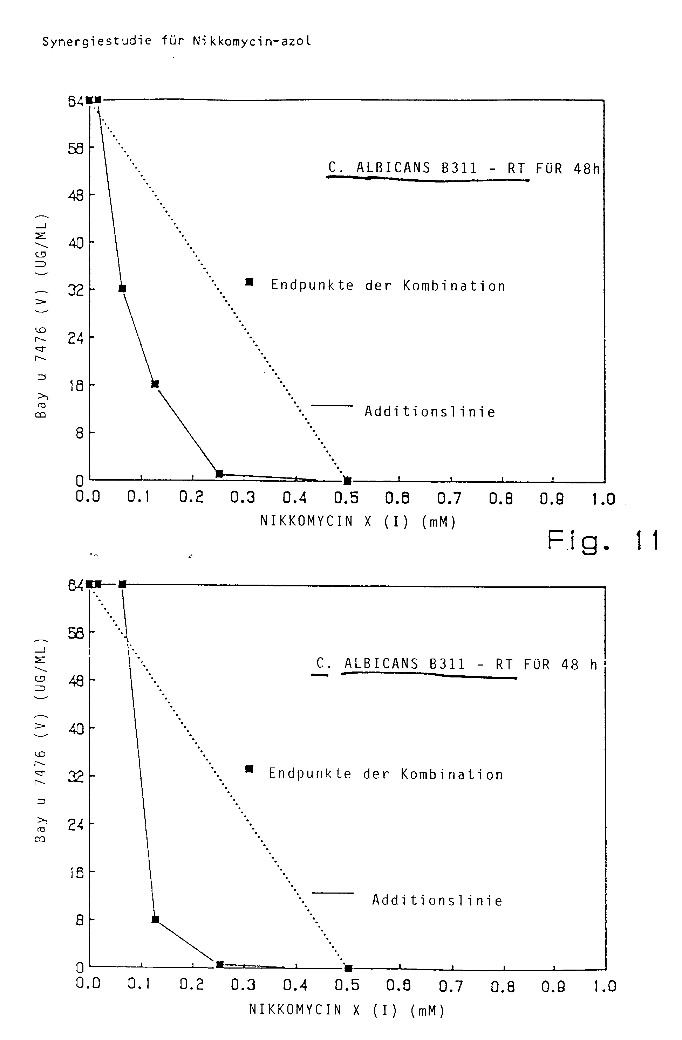

Die Ergebnisse der Versuche sind in den Fig. 3 bis 11 dargestellt. Man erkennt, daß für alle Azole die Zugabe von Nikkomycinen eine Herabsetzung der Endpunkte im Vergleich zu den Endpunkten der einzelnen Mittel selbst bewirkt. Zum Beispiel zeigt Fig. 3 die Endpunkte für Clotrimazol alleine bei 2 bis 4 µg/ml und 0,25 bis 0,5 mMol für die Nikkomycine. Überraschenderweise wurde mit den Konzentrationen der in diesem Beispiel verwendeten Kombinationen kein Wachstum festgestellt, einschließlich des Minimums von 0,06 µg Azol-0,02 mMol Nikkomycin. Somit wurde der Endpunkt für die Kombination auf ein hinsichtlich des Azolendpunktes 50-faches Minimum reduziert, was einen deutlichen Beweis für die Synergie dieser zwei Klassen an Wirkstoffen darstellt.

Wirksamkeit von Nikkomycin X und Zim Tiermodell von Murine Candidosis.

Gezüchtete $CFW_1$-Mäuse wurden intravenös mit 6 x $10^5$ cfu von C. albicans infiziert. Die orale Therapie wurde gleichzeitig mit der Infektion gestartet. Die Mitteln wurden einzeln mit Nährnadeln in Volumina von 0,5 ml ausgegeben.

Im Modell der septischen Murine candidosis, das häufig zum Testen von Azolen verwendet wird, wird die Behandlung der Tiere gleichzeitig mit der Infektion gestartet indem man 2 x 100 mg/kg beider Zubereitungen am Tag der Infektion verabreicht. Diese Verabreichungsweise erzeugt nur eine leichte Verzögerung in der letalen Kinetik (Tabelle 1).

| Präparat | Dosis/Tag in mg/kg | Zahl der überlebenden Tiere in den folgenden Tagen nach der Infektion 0 | 3 | 6 |
|---|---|---|---|---|
| Kontrolle | --- | 20 | 12 | 1 |
| Nikkomycin X | 2 x 100 | 10 | 10 | 3 |
| Nikkomycin Z | 2 x 100 | 10 | 6 | 5 |

Tabelle 1

Wirkung von Nikkomycin X und Z bei septischer Murine candidosis nach oraler Verabreichung

Eine verbesserte Wirksamkeit kann erreicht werden, wenn man den Behandlungszeitraum auf 3 Tage (Fig. 1) ausdehnt. Unter diesen Testbedingungen ist die Wirksamkeit von 100 mg/kg Nikkomycin Z vergleichbar zu der von 25 mg/kg Ketoconazol oder Vibunazol.

Ein beträchtlicher Anstieg in der Wirksamkeit von Nikkomycin Z wird alleine bei einer 14-tägigen Behandlung (Fig. 2) erreicht. In diesem Schema ist eine tägliche Dosis von 2 x 10 mg/kg wirksamer als 2 x 100 mg/kg, verabreicht über 3 Tage (Fig. 1,2).

Die Fig. 3 bis 11 zeigen Synergiestudien hinsichtlich des antifungalen Tests von wirksamen Nikkomycin-Azol- Zusammensetzungen.

Um die Eignung von Nikkomycinen und Azolen hinsichtlich der synergetischen Wirksamkeit zu testen, werden schachbrettartige in vitro-Versuche durchgeführt, in denen die Hefe Candida albicans eingesetzt wurde.

Die Beispiele haben lediglich erläuternden Charakter und zeigen nicht die Anwendungsgrenzen der Erfindung.

**Patentansprüche**

**Patentansprüche für folgende Vertragsstaaten : AT, BE, CH, DE, FR, GB, IT, LI, NL, SE**

1. Antimykotische Zusammensetzungen, die eine antimykotisch wirksame Menge eines Nikkomycins, ausgewählt aus Nikkomycin Z und Nikkomycin X und 1-(4-Chlorphenyl)-2-methyl-2-methoximino-methyl-1-(1,2,4-triazol-1-yl-methyl)-1-propanol, 1-(4-Chlorphenoxy)-3,3-dimethyl-2-(1,2,4-triazol-1-yl-methyl)-butan-2-ol, 1-(4-Fluorphenyl)-1-(1-methylsulfinyl-1-cyclopropyl)-(1,2,4-triazol-1-yl-methyl)-methanol, Miconazol, Ketoconazol, Itraconazol, Fluconazol, Clotrimazol und/oder Bifonazol der deren Säureadditionssalze sowie übliche pharmazeutische Hilfsstoffe enthalten.

2. Verwendung von antimykotischen Zusammensetzungen, enthaltend eine antimykotisch wirksame Menge eines Nikkomycins, ausgewählt aus Nikkomycin Z und Nikkomycin X und 1-(4-Chlorphenyl)-2-methyl-2-methoximino-methyl-1-(1,2,4-triazol-1-yl-methyl)-1-propanol, 1-(4-Chlorphenoxy)-3,3-dimethyl-2-(1,2,4-triazol-1-y 1-methyl)-butan-2-ol, 1-(4-Fluorphenyl)-1-(1-methylsulfinyl-1cyclopropyl)-(1,2,4-triazol-1-yl-methyl)-methanol, Miconazol, Ketoconazol, Itraconazol, Fluconazol, Clotrimazol und/-oder Bifonazol der deren Säureadditionssalze bei der Herstellung von antimykotischen Mitteln.

3. Verfahren zur Herstellung von antimykotischen zusammensetzungen gemäß Anspruch 1, dadurch gekennzeichnet, daß eine antimykotisch wirksame Menge eines Nikkomycins, ausgewählt aus Nikkomycin Z und Nikkomycin X in Kombination mit mindestens einem Azolderivat gemäß Anspruch 1 mit einem festen oder flüssigen Streckmittel und/oder -träger oder anderen, für die Herstellung von pharmazeutischen Zusammensetzungen geeigneten Hilfsmitteln vermischt wird.

**Patentansprüche für folgenden Vertragsstaat : GR**

**1.** Antimykotisches Mittel, bestehend aus einer antimykotisch wirksamen Menge eines Nikkomycins, ausgewählt aus Nikkomycin Z und Nikkomycin X und 1-(4-Chlorphenyl)-2-methyl-2-methoximino-methyl-1-(1,2,4-triazol-1-yl-methyl)-1-propanol, 1-(4-Chlorphenoxy)-3,3-dimethyl-2-(1,2,4-triazol-1-yl-methyl)-butan-2-ol, 1-(4-Fluorphenyl)-1-(1-methylsulfinyl-1cyclopropyl)-(1,2,4-triazol-1-yl-methyl)-methanol, Miconazol, Ketoconazol, Itraconazol, Fluconazol, Clotrimazol und/oder Bifonazol oder deren Säureadditionssalze sowie üblichen pharmazeutischen Hilfsstoffen.

**2.** Verfahren zur Herstellung von antimykotischen Mitteln gemäß Anspruch 1, dadurch gekennzeichnet, daß eine antimykotisch wirksame Menge eines Nikkomycins, ausgewählt aus Nikkomycin Z und Nikkomycin X in Kombination mit mindestens einem Azolderivat gemäß Anspruch 1 mit einem festen oder flüssigen Streckmittel und/oder -träger oder anderen, für die Herstellung von pharmazeutischen Zusammensetzungen geeigneten Hilfsmitteln vermischt wird.

**Patentanspruch für folgenden Vertragsstaat : ES**

**1.** Verfahren zur Herstellung von antimykotischen Zusammensetzungen, enthaltend eine antimykotisch wirksame Menge eines Nikkomycins, ausgewählt aus Nikkomycin Z und Nikkomycin X und 1-(4-Chlorphenyl)-2-methyl-2-methoximino-methyl-1-(1,2,4-triazol-1-yl-methyl)-1-propanol, 1-(4-Chlorphenoxy)-3,3-dimethyl-2-(1,2,4-triazol-1-yl-methyl)-butan-2-ol, 1-(4-Fluorphenyl)-1-(1-methylsulfinyl-1-cyclopropyl)-(1,2,4-triazol-1-yl-methyl)-methanol, Miconazol, Ketoconazol, Itraconazol, Fluconazol, Clotrimazol und/oder Bifonazol, oder deren Säureadditionssalze in welchem die Wirkstoffkombination mit einem festen oder flüssigen Streckmittel und/oder -träger oder anderen, für die Herstellung von pharmazeutischen Zusammensetzungen geeigneten Hilfsmitteln vermischt wird.

## Claims

**Claims for the following Contracting States : AT, BE, CH, DE, FR, GB, IT, LI, NL, SE**

**1.** Antimycotic compositions, which contain an antimycotically active amount of a nikkomycin selected from nikkomycin Z and nikkomycin X and 1-(4-chlorophenyl)-2-methyl-2-methoximino-methyl-1-(1,2,4-triazol-1-yl-methyl)-1-propanol, 1-(4-chlorophenoxy)-3,3-dimethyl-2-(1,2,4-triazol-1-yl-methyl)-butan-2-ol, 1-(4-fluorophenyl)-1-(1-methylsulphinyl-1-cyclopropyl)-(1,2,4-triazol-1-yl-methyl)-methanol, miconazole, ketoconazole, itraconazole, fluconazole, clotrimazole and/or bifonazole or their acid addition salts and customary pharmaceutical auxiliaries.

**2.** Use of antimycotic compositions containing an antimycotically active amount of a nikkomycin selected from nikkomycin Z and nikkomycin X and 1-(4-chlorophenyl)-2-methyl-2-methoximino-methyl-1-(1,2,4-triazol-1-yl-methyl)-1-propanol, 1-(4-chlorophenoxy)-3,3-dimethyl-2-(1,2,4-triazol-1-yl-methyl)-butan-2-ol, 1-(4-fluorophenyl)-1-(1-methylsulphinyl-1-cyclopropyl)-(1,2,4-triazol-1-yl-methyl)-methanol, miconazole, ketoconazole, itraconazole, fluconazole, clotrimazole and/or bifonazole or their acid addition salts in the preparation of antimycotic agents.

**3.** Process for the production of antimycotic compositions according to Claim 1, characterised in that an antimycotically active amount of a nikkomycin selected from nikkomycin Z and nikkomycin X in combination with at least one azole derivative according to Claim 1 is mixed with a solid or liquid extender and/or excipient or other auxiliaries suitable for the production of pharmaceutical compositions.

**Claims for the following Contracting State : GR**

**1.** Antimycotic agent, consisting of an antimycotically active amount of a nikkomycin selected from nikkomycin Z and nikkomycin X and 1-(4-chlorophenyl)-2-methyl-2-methoximino-methyl-1-(1,2,4-triazol-1-yl-methyl)-1-propanol, 1-(4-chlorophenoxy)-3,3-dimethyl-2-(1,2,4-triazol-1-yl-methyl)-butan-2-ol, 1-(4-fluorophenyl)-1-(1-methylsulphinyl-1-cyclopropyl)-(1,2,4-triazol-1-yl-methyl)-methanol, miconazole, ketoconazole, itraconazole, fluconazole, clotrimazole and/or bifonazole or their acid addition salts and customary pharmaceutical auxiliaries.

**2.** Process for the production of antimycotic agents according to Claim 1, characterised in that an antimycotically active amount of a nikkomycin selected from nikkomycin Z and nikkomycin X in

combination with at least one azole derivative according to Claim 1 is mixed with a solid or liquid extender and/or excipient or other auxiliaries suitable for the production of pharmaceutical compositions.

**Claim for the following Contracting State : ES**

1. Process for the production of antimycotic compositions containing an antimycotically active amount of a nikkomycin selected from nikkomycin Z and nikkomycin X and 1-(4-chlorophenyl)-2-methyl-2-methoximino-methyl-1-(1,2,4-triazol-1-yl-methyl)-1-propanol, 1-(4-chlorophenoxy)-3,3-dimethyl-2-(1,2,4-triazol-1-yl-methyl)-butan-2-ol, 1-(4-fluorophenyl)-1-(1-methylsulphinyl-1-cyclopropyl)-(1,2,4-triazol-1-yl-methyl)-methanol, miconazole, ketoconazole, itraconazole, fluconazole, clotrimazole and/or bifonazole or their acid addition salts, in which the active compound combination is mixed with a solid or liquid extender and/or excipient or other auxiliaries suitable for the preparation of pharmaceutical compositions.

**Revendications**
**Revendications pour les Etats contractants suivants : AT, BE, CH, FR, GB, IT, LI, NL, SE**

1. Compositions antimycosiques, qui contiennent une quantité, douée d'activité antimycosique, d'une nikkomycine, choisie entre la nikkomycine Z et la nikkomycine X, et du 1-(4-chlorophényl)-2-méthyl-2-méthoximino-méthyl-1-(1,2,4-triazole-1-yl-méthyl)-1-propanol, du 1-(4-chlorophénoxy)-3,3-diméthyl-2-(1,2,4-triazole-1-yl-méthyl)-butane-2-ol, du 1-(4-fluorophényl)-1-(1-méthylsulfinyl-1-cyclopropyl)-(1,2,4-triazole-1-yl-méthyl)-méthanol, du miconazole, du kétoconazole, de l'itraconazole, du fluconazole, du clotrimazole et/ou du bifonazole ou leurs sels d'addition d'acides, ainsi que des substances auxiliaires pharmaceutiques classiques.

2. Utilisation de compositions antimycosiques, contenant une quantité à effet antimycosique d'une nikkomycine, choisie entre la nikkomycine Z et la nikkomycine X, et du 1-(4-chlorophényl)-2-méthyl-2-méthoximino-méthyl-1-(1,2,4-triazole-1-yl-méthyl)-1-propanol, du 1-(4-chlorophénoxy)-3,3-diméthyl-2-(1,2,4-triazole-1-yl-méthyl)-butane-2-ol, du 1-(4-fluorophényl)-1-(1-méthylsulfinyl-1-cyclopropyl)-(1,2,4-triazole-1-yl-méthyl)-méthanol, du miconazole, du kétoconazole, de l'itraconazole, du fluconazole, du clotrimazole et/ou du bifonazole ou leurs sels d'addition d'acides, dans la préparation d'agents antimycosiques.

3. Procédé de préparation de compositions antimycosiques suivant la revendication 1, caractérisé en ce qu'une quantité à effet antimycosique d'une nikkomycine, choisie entre la nikkomycine Z et la nikkomycine X, en association avec au moins un dérivé azolique suivant la revendication 1, est mélangée avec un diluant et/ou un support solide ou liquide ou d'autres substances auxiliaires qui conviennent pour la préparation de compositions pharmaceutiques.

**Revendications pour l'Etat contractant suivant : GR**

1. Agent antimycosique, constitué d'une quantité à effet antimycosique d'une nikkomycine, choisie entre la nikkomycine Z et la nikkomycine X, et de 1-(4-chlorophényl)-2-méthyl-2-méthoximino-méthyl-1-(1,2,4-triazole-1-yl-méthyl)-1-propanol, de 1-(4-chlorophénoxy)-3,3-diméthyl-2-(1,2,4-triazole-1-yl-méthyl)-butane-2-ol, de 1-(4-fluorophényl)-1-(1-méthylsulfinyl-1-cyclopropyl)-(1,2,4-triazole-1-yl-méthyl)-méthanol, de miconazole, de kétoconazole, d'itraconazole, de fluconazole, de clotrimazole et/ou de bifonazole ou de leurs sels d'addition d'acides, ainsi que de substances auxiliaires pharmaceutiques classiques.

2. Procédé de préparation d'agents antimycosiques suivant la revendication 1, caractérisé en ce qu'on mélange une quantité à effet antimycosique d'une nikkomycine, choisie entre la nikkomycine Z et la nikkomycine X, en association avec au moins un dérivé azolique suivant la revendication 1, avec un diluant et/ou un support solide ou liquide ou d'autres substances auxiliaires qui conviennent pour la préparation de compositions pharmaceutiques.

**Revendication pour l'Etat contractant suivant : ES**

**1.** Procédé de production de préparations antimycosiques, contenant une quantité à effet antimycosique d'une nikkomycine, choisie entre la nikkomycine Z et la nikkomycine X, et du 1-(4-chlorophényl)-2-méthyl-2-méthoximino-méthyl-1-(1,2,4-triazole-1-yl-méthyl)-1-propanol, du 1-(4-chlorophénoxy)-3,3-diméthyl-2-(1,2,4-triazole-1-yl-méthyl)-butane-2-ol, du 1-(4-fluorophényl)-1-(1-méthylsulfinyl-1-cyclopropyl)-(1,2,4-triazole-1-yl-méthyl)-méthanol, du miconazole, du kétoconazole, de l'itraconazole, du fluconazole, du clotrimazole et/ou du bifonazole ou leurs sels d'addition d'acides, dans lequel l'association de substances actives est mélangée avec un diluant et/ou un support solide ou liquide ou d'autres substances auxiliaires qui conviennent pour la préparation de compositions pharmaceutiques.

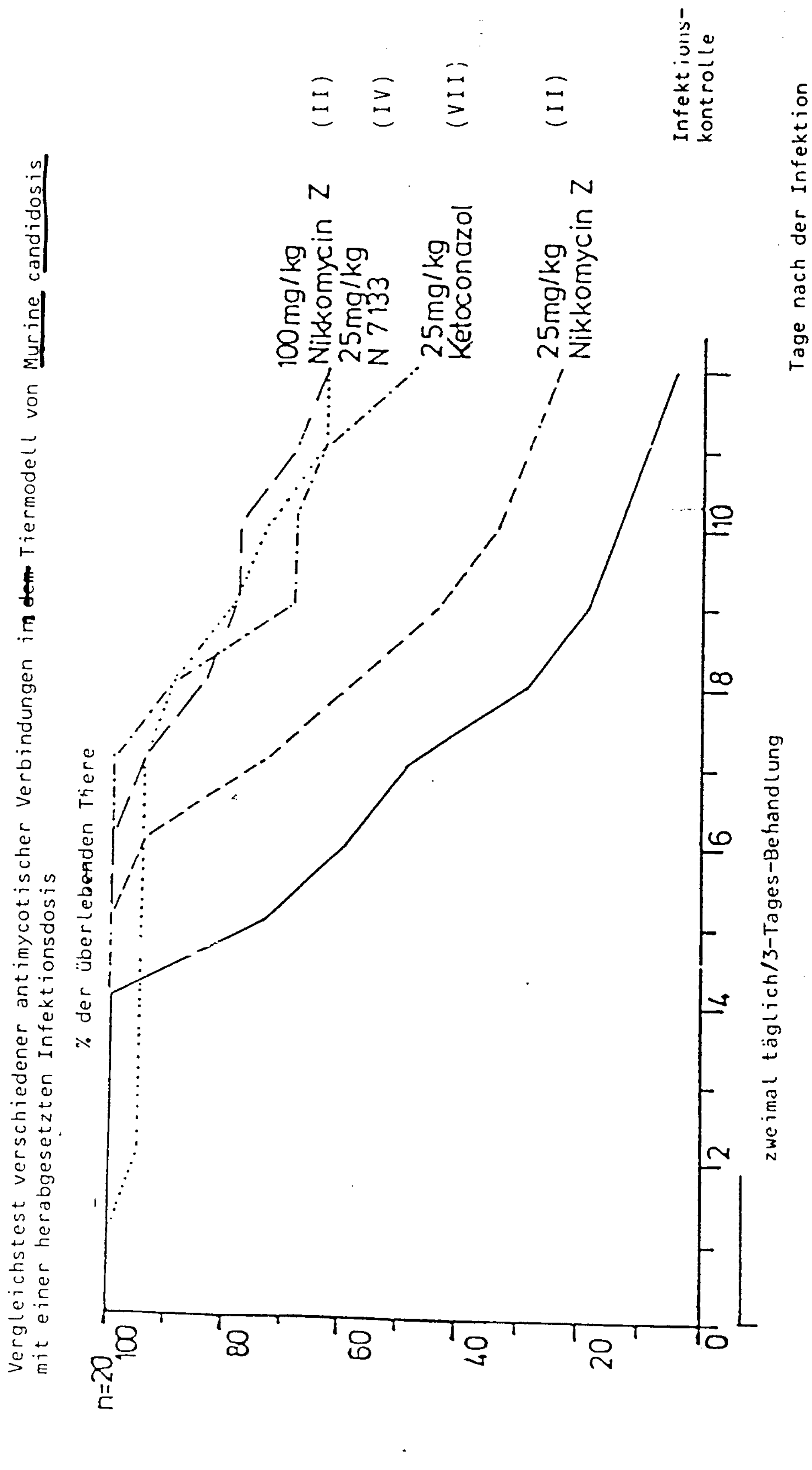
Vergleichstest verschiedener antimycotischer Verbindungen in Tiermodell von Murine candidosis
mit einer herabgesetzten Infektionsdosis
% der überlebenden Tiere
n=20
100
80
60
40
20
0
2
4
6
8
10
100mg/kg Nikkomycin Z
(II)
25mg/kg N 7133
(IV)
25mg/kg Ketoconazol
(VII)
25mg/kg Nikkomycin Z
(II)
Infektions-kontrolle
Tage nach der Infektion
zweimal täglich/3-Tages-Behandlung

FIG.1

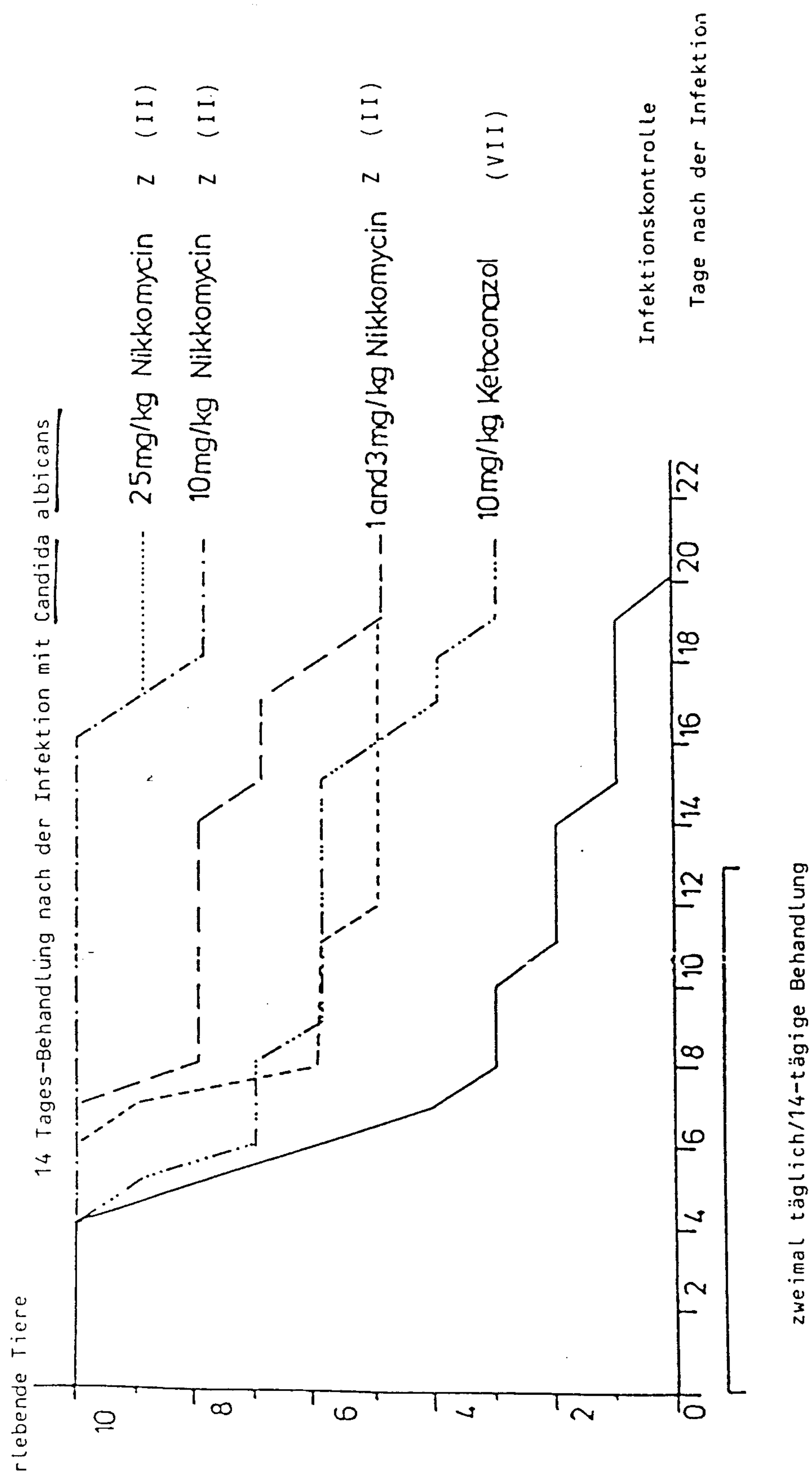
14 Tages-Behandlung nach der Infektion mit Candida albicans
25mg/kg Nikkomycin Z (II)
10mg/kg Nikkomycin Z (II)
1 and 3 mg/kg Nikkomycin Z (II)
10mg/kg Ketoconazol (VII)
Infektionskontrolle
Tage nach der Infektion
überlebende Tiere
10
8
6
4
2
0
2
4
6
8
10
12
14
16
18
20
22
zweimal täglich/14-tägige Behandlung

FIG. 2

Synergiestudie für Nikkomycin-azol

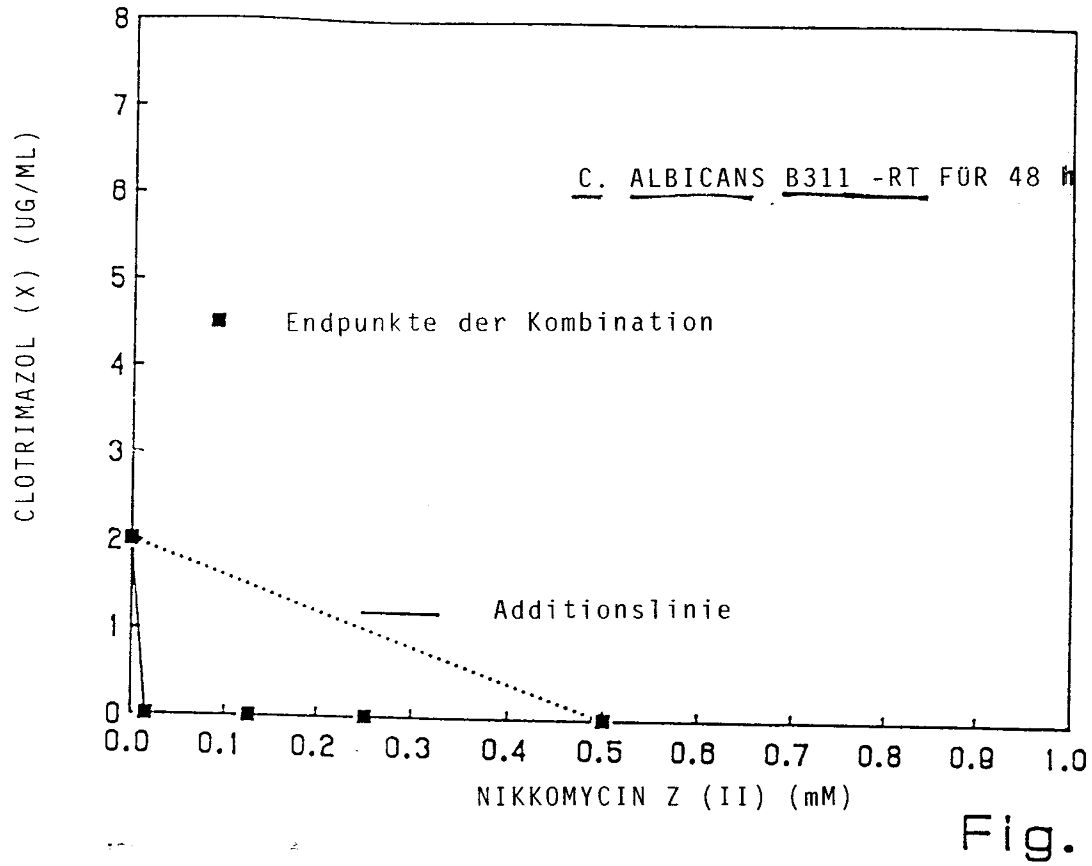


Fig. 3

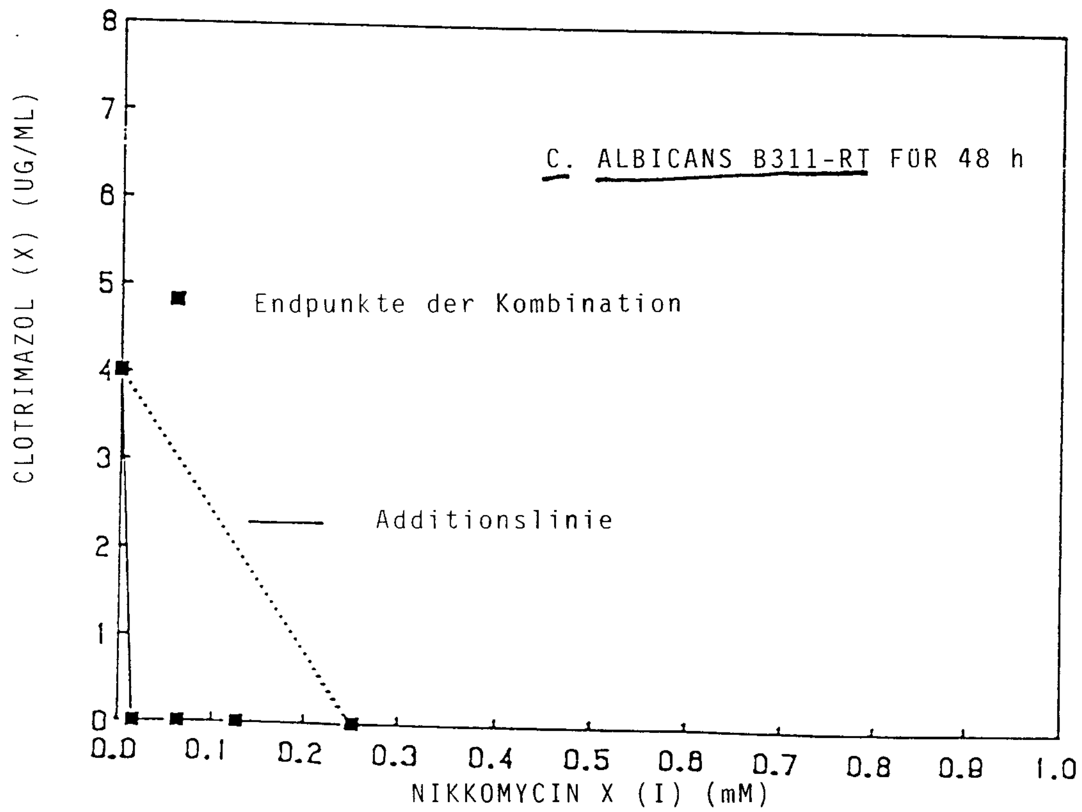

Synergiestudie für Nikkomycin-azol

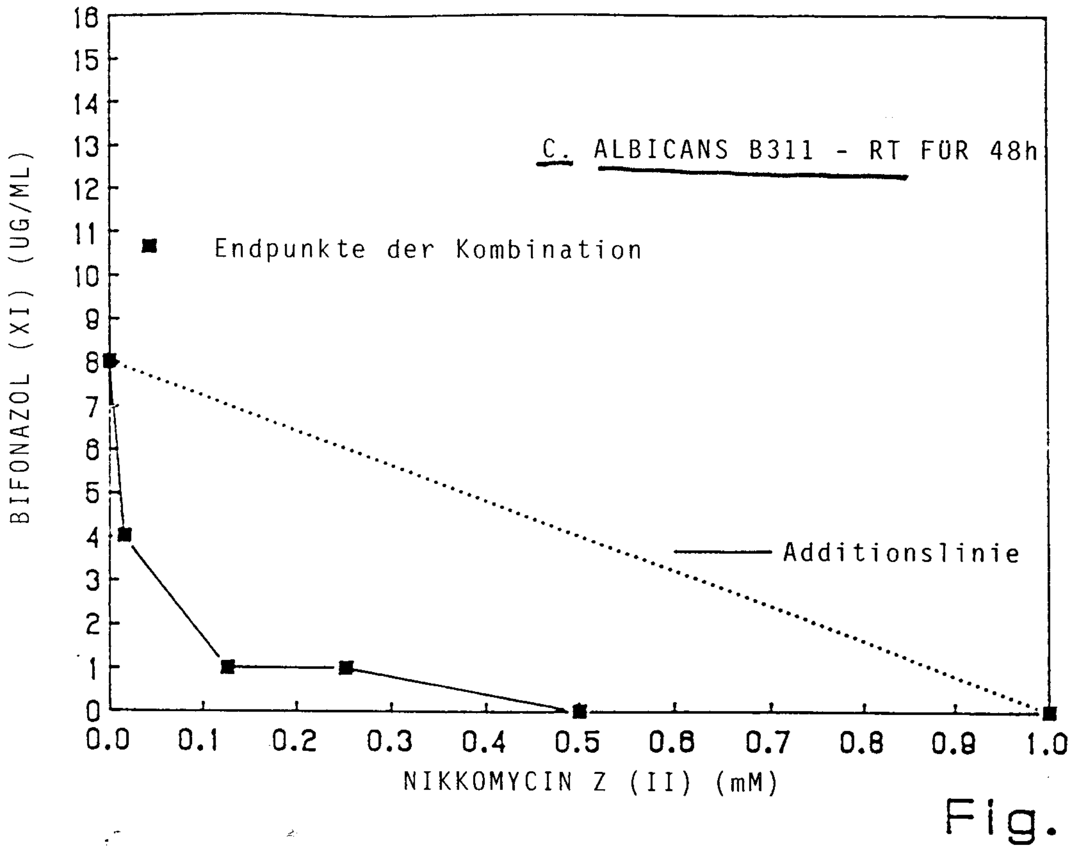


Fig. 4

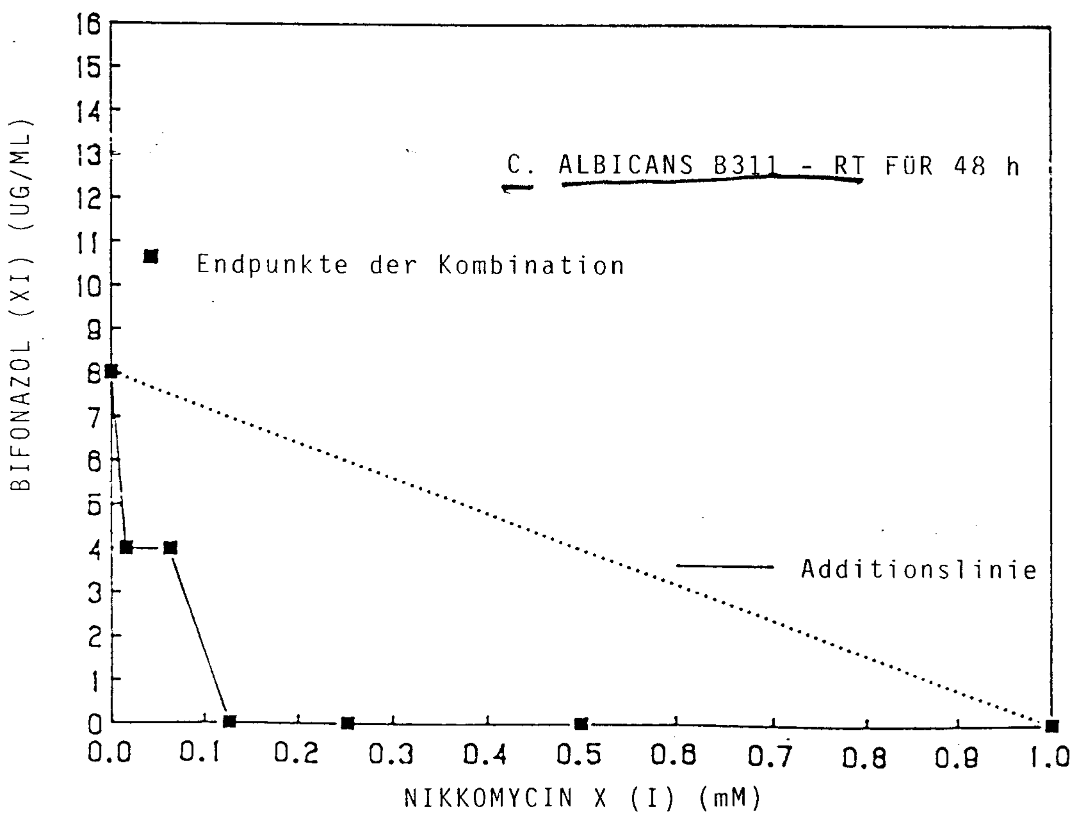

Synergiestudie für Nikkomacin-azol

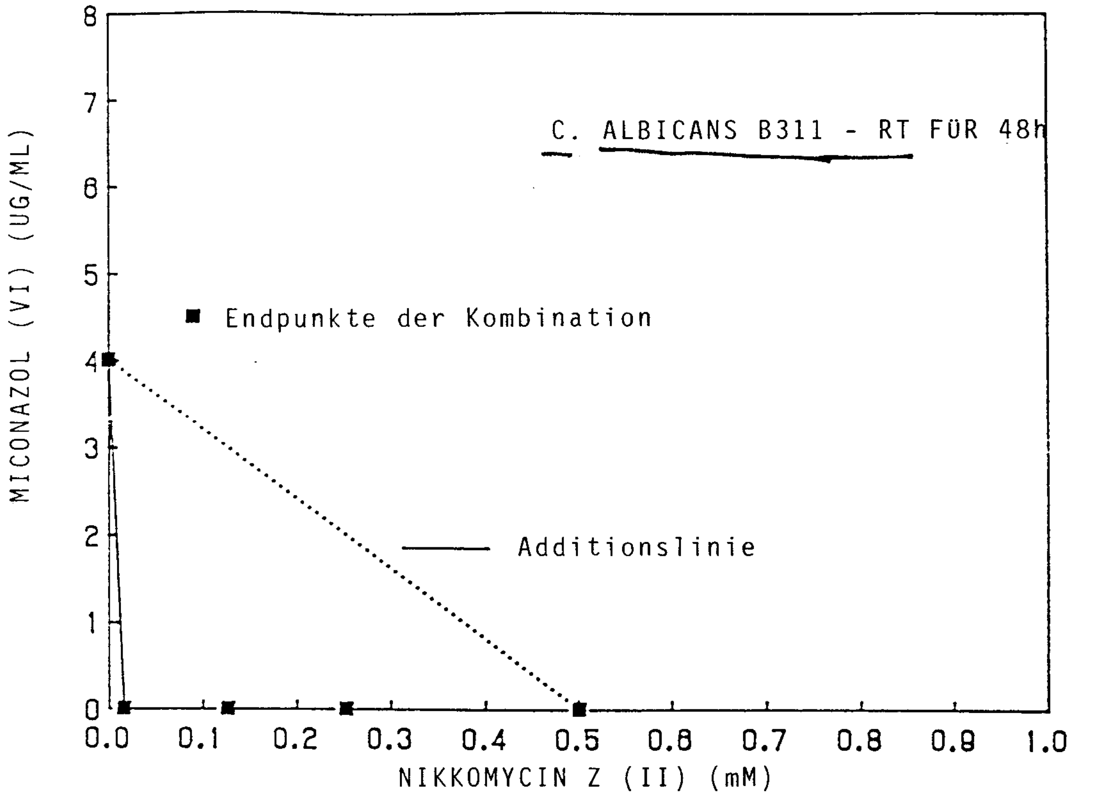


Fig. 5

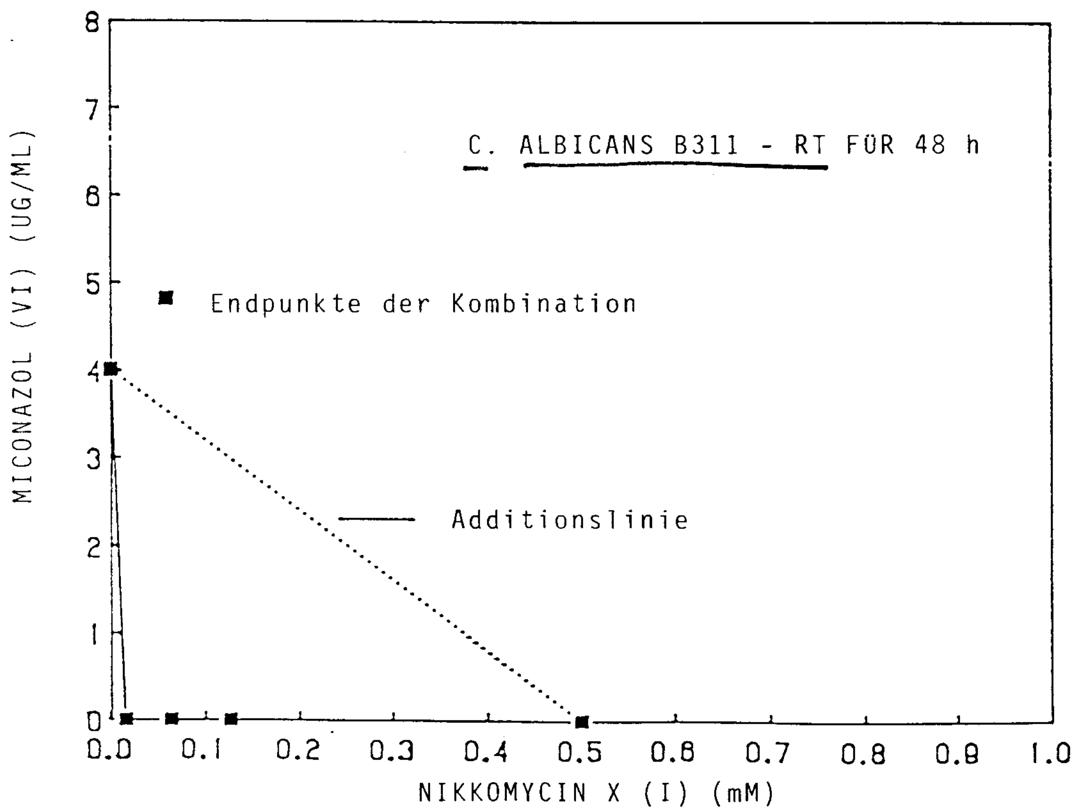

Synergiestudie für Nikkomycin-azol

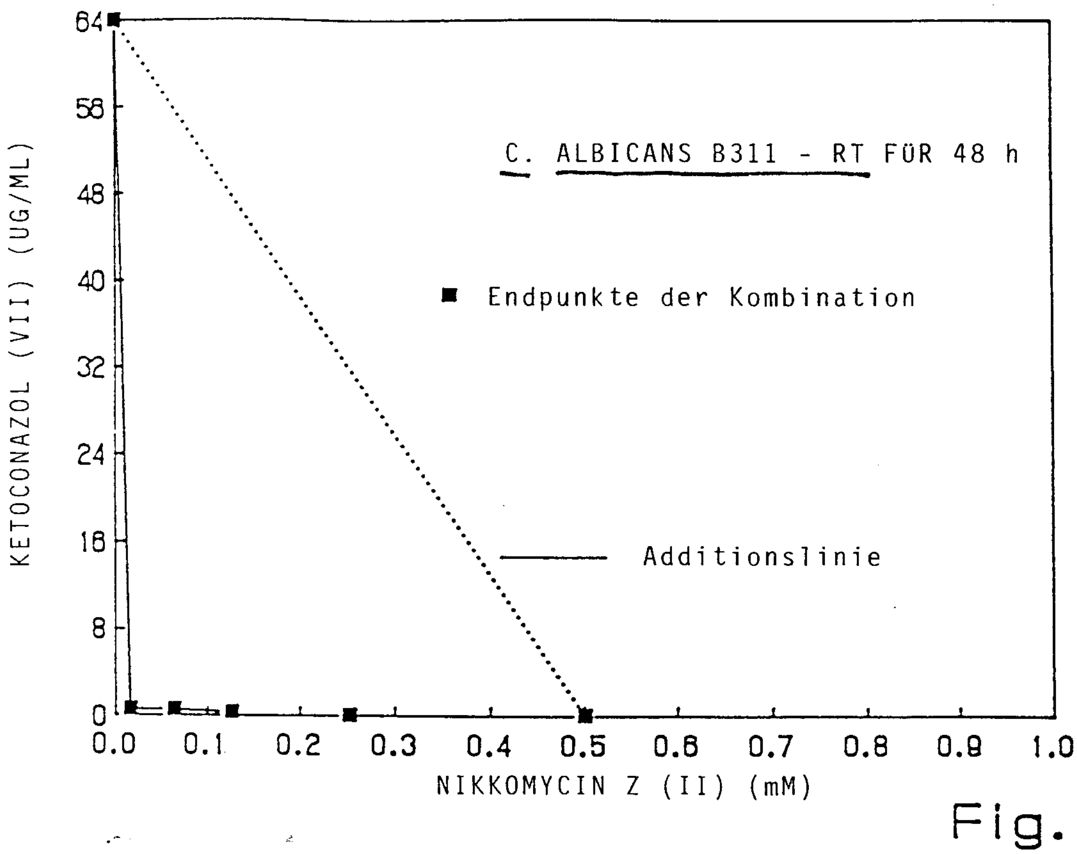


Fig. 6

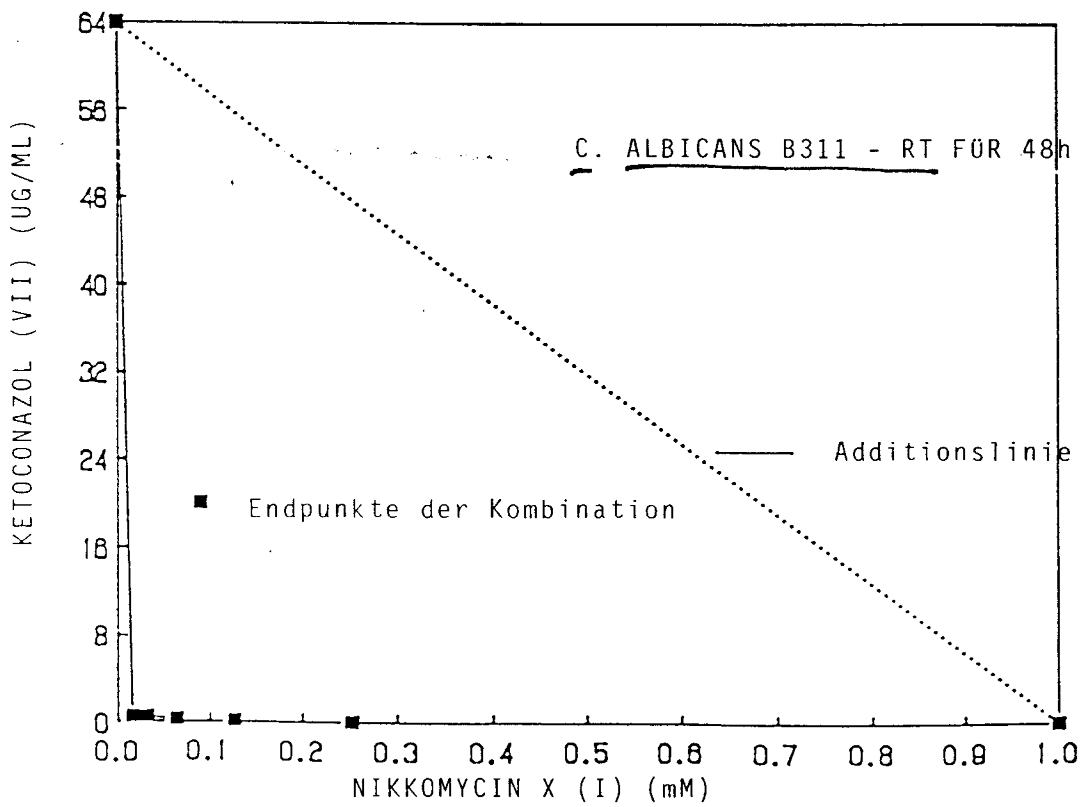

Synergiestudie für Nikkomycin-azol

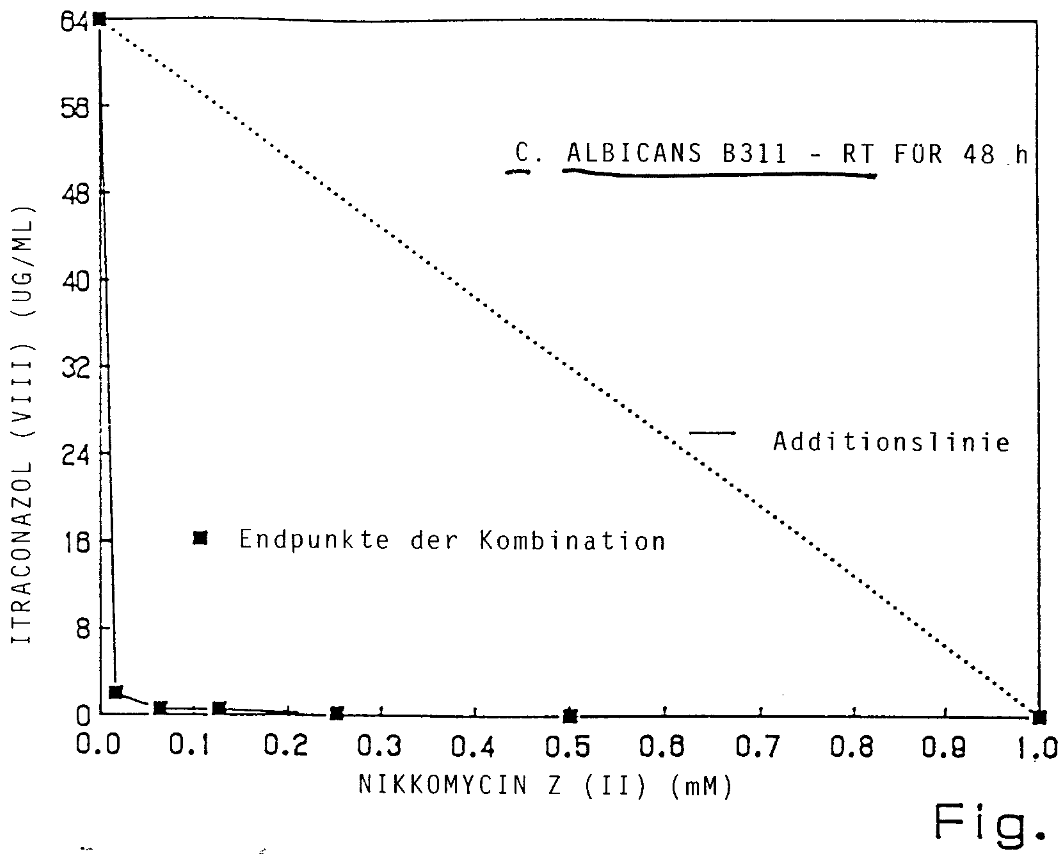


Fig. 7

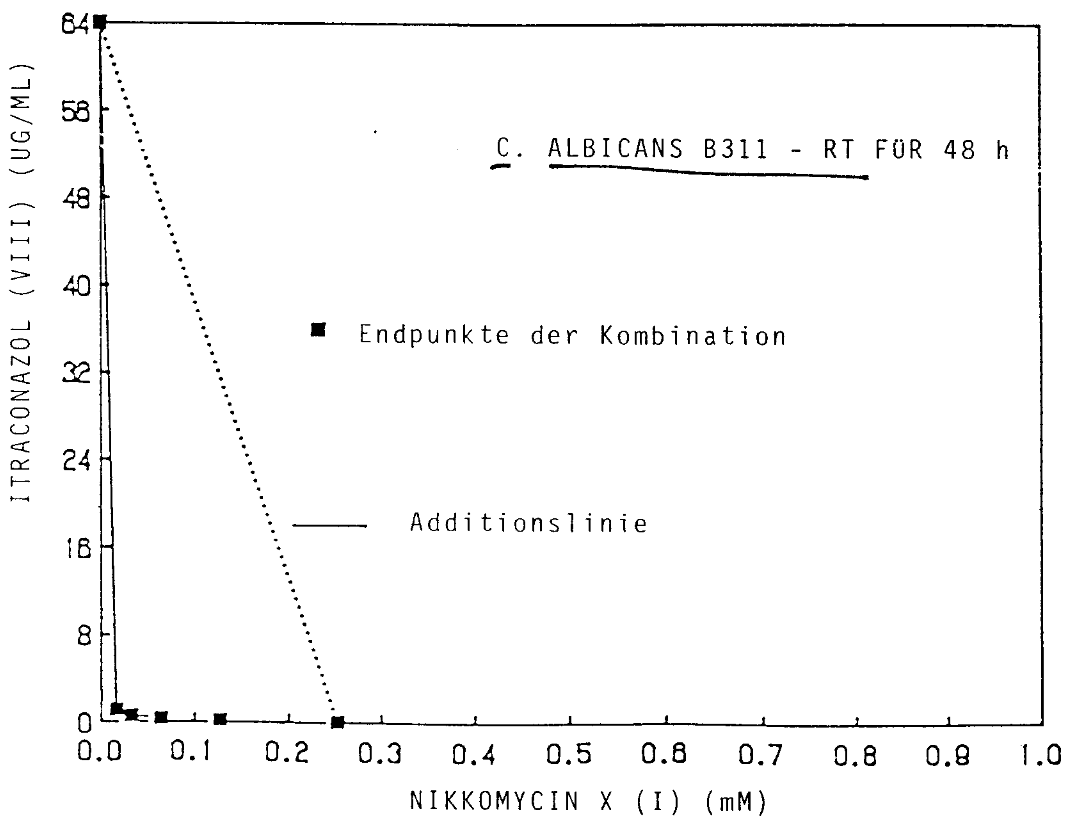

Synergiestudie für Nikkomycin-azol

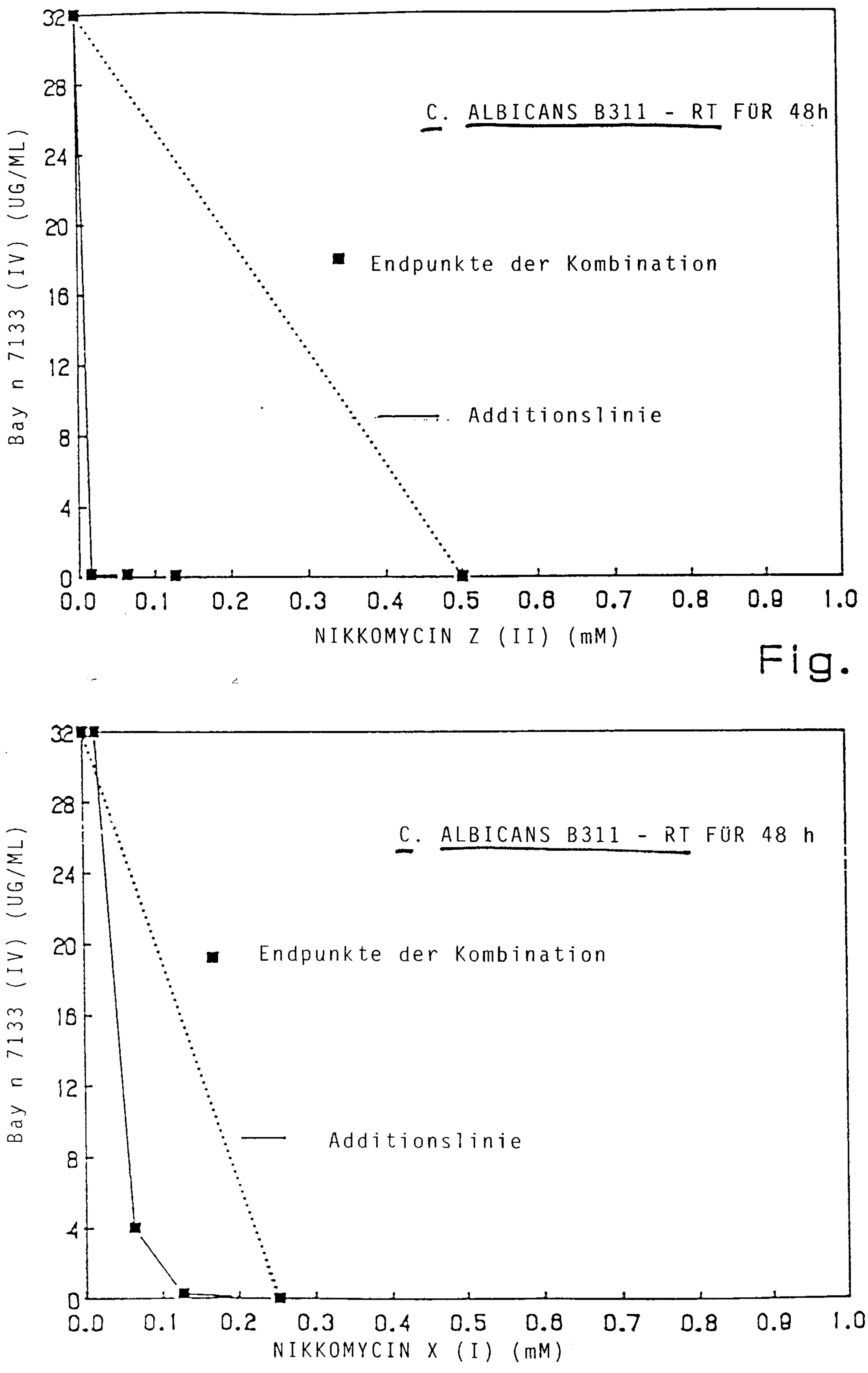


Fig. 8

Synergiestudie für Nikkomycin-azol

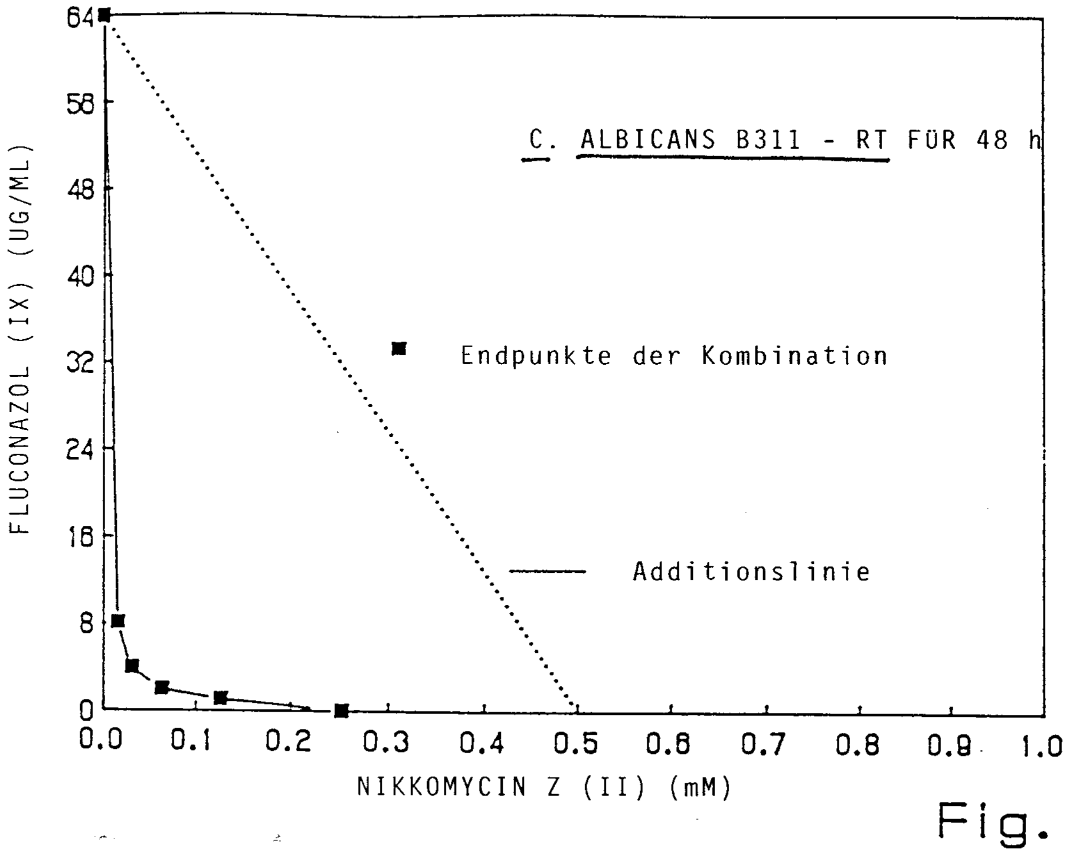


Fig. 9

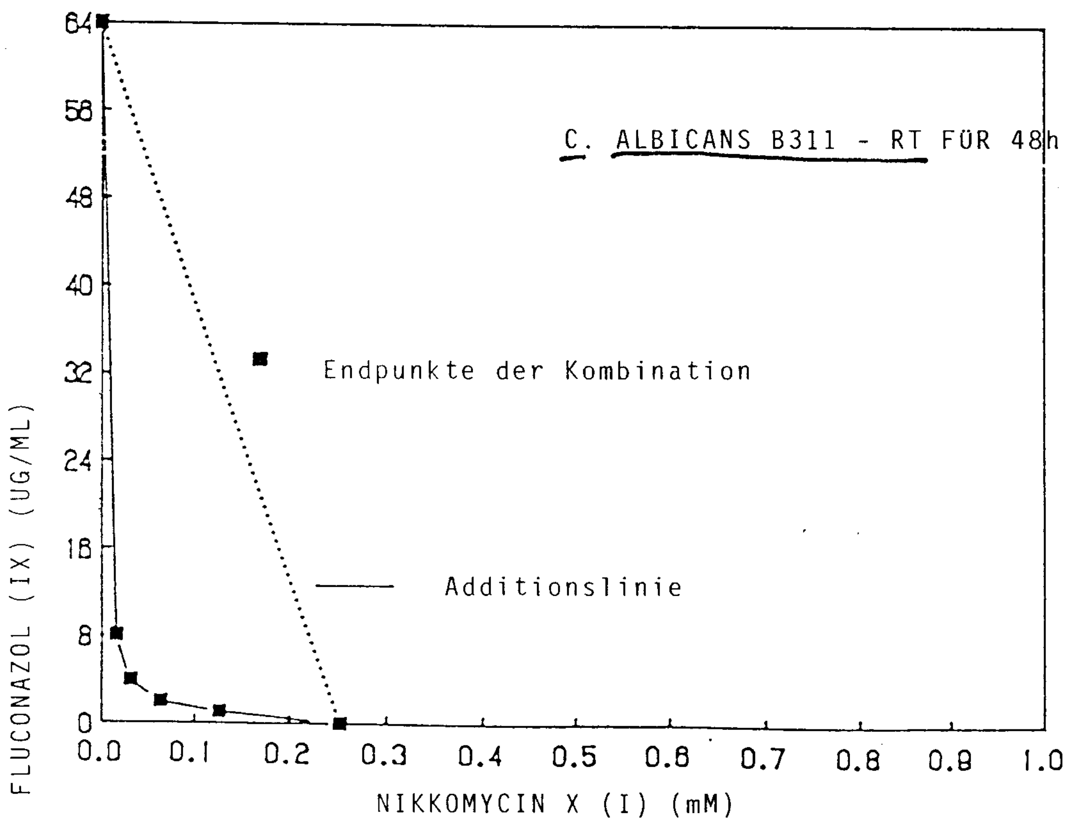

Synergiestudie für Nikkomycin-azol

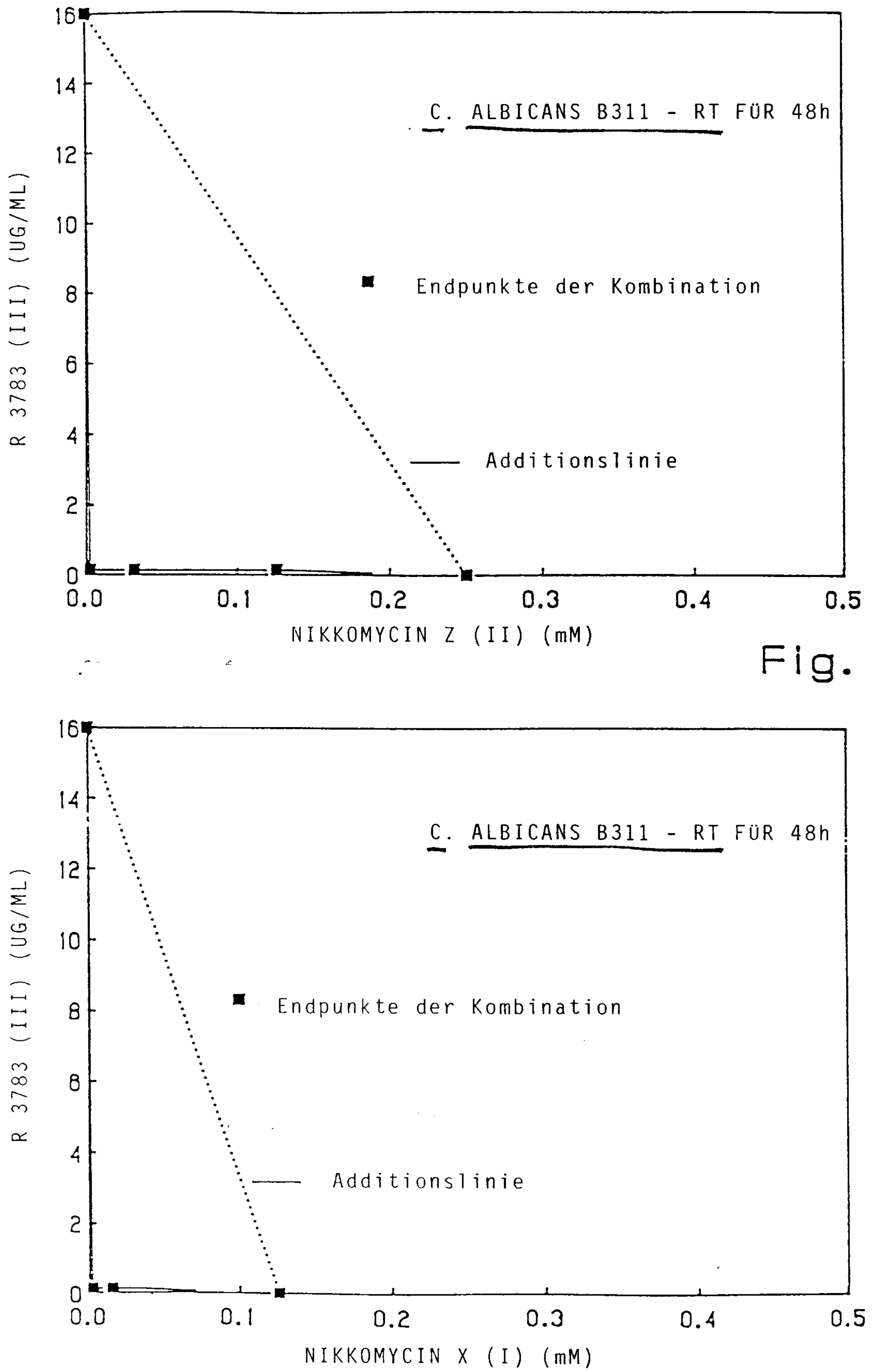


Fig. 10

Synergiestudie für Nikkomycin-azol

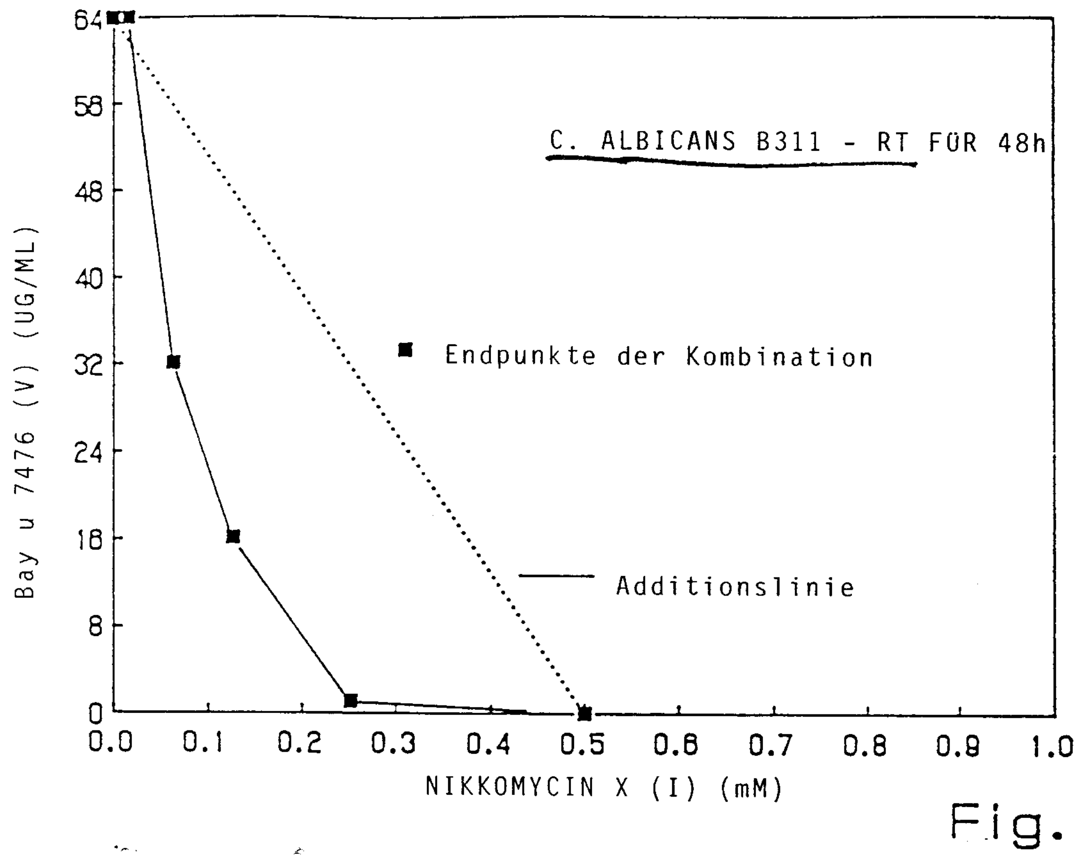


Fig. 11